# EUROPÄISCHE PATENTANMELDUNG

(11) **EP 1 922 998 A1**
(43) Veröffentlichungstag der Anmeldung: **21.05.2008**
(21) Anmeldenummer: 07120718.7
(22) Anmeldetag: 14.11.2007
(51) Int. Cl.: A61B 5/1455

(54) **Vorrichtung und Verfahren zum nicht-invasiven, optischen Erfassen von chemischen und physikalischen Blutwerten und Körperinhaltsstoffen**

(30) Priorität: 20.11.2006 DE 102006054556
(71) Anmelder: Zimmer MedizinSysteme GmbH, 89231 Neu-Ulm (DE)
(72) Erfinder: Bussek, Karlheinz, 86459, Deubach (DE)
(74) Vertreter: Peckmann, Ralf

(57) **Zusammenfassung**

Die vorliegende Erfindung bezieht sich allgemein auf eine Vorrichtung und ein Verfahren zur nicht-invasiven Bestimmung von Körperinhaltsstoffen sowie der Validierung von chemischen und physikalischen Kennwerten von Blut oder anderen Körperflüssigkeiten. Insbesondere bezieht sich die Erfindung auf eine nicht-invasive Bestimmung des Blutdrucks in Verbindung mit weiteren Blutwerten von arteriellem Blut unter Verwendung von optischen Verfahren an einer Patientenschnittstelle (23), die eine erste Vorrichtung (1) in Form eines durchstimmbaren optischen Senders (3) und eine zweite Vorrichtung (2) in Form eines durchstimmbaren optischen Empfängers (4) umfaßt, dadurch gekennzeichnet, dass der optische Sender (3) und der optische Empfänger (4) mittels einer Steuer- oder Regelungseinheit (5) zueinander synchronisiert sind.

## Beschreibung

Die vorliegende Erfindung bezieht sich allgemein auf eine nicht-invasive Bestimmung von Körperinhaltsstoffen sowie eine Validierung von chemischen und physikalischen Kennwerten von Blut oder anderen Körperflüssigkeiten. Insbesondere bezieht sich die Erfindung auf eine nicht-invasive Bestimmung des Blutdrucks in Verbindung mit weiteren Blutwerten von arteriellem Blut unter Verwendung von optischen Verfahren.

Die Ermittlung von chemischen Inhaltsstoffen sowie der Messung des Blutdrucks ist durch diverse Verfahren hinreichend bekannt und die diversen Verfahren sind im Stand der Technik beschrieben. Insbesondere bekannt sind Blutdruckmessgeräte zur Bestimmung des arteriellen Blutdrucks. Das Herz ist die treibende Kraft des Blutdrucks. Während der Austreibungsphase des Blutes (Systole) wird das ausgetriebene Blutvolumen ausgeworfen und in der Aorta gespeichert und danach in der Entspannungsphase (Diastole) an das nachfolgende Gefäßsystem wieder abgegeben. Der Druck bzw. der arterielle Blutdruck, der dabei entsteht wird durch diverse Faktoren bestimmt und beeinflußt. Dies sind im Wesentlichen das Volumen des Herzens und die Schlagkraft, also die Herzleistung, die Elastizität der Arterien, der periphere Strömungswiderstand der Blutgefäße und das Volumen des Blutes. Heute übliche Meßverfahren lassen sich prinzipiell in zwei Gruppen einteilen:
a) kontinuierliches Verfahren und
b) diskontinuierliches Verfahren.

Bei den diskontinuierlichen Verfahren handelt es sich in der Regel um Verfahren, welche auf Riva-Rocci zurückgehen und mittels einer Membran und einer geeigneten Messmanschette den Druck bzw. die Druckänderung und die damit verbundenen Korotkow-Töne erfassen, die entweder über ein Stethoskop oder mittels alternativer Verfahren ermittelt werden können. Dabei werden die unterschiedlichen Töne und die Veränderung dieser Töne dem systolischen und diastolischen Blutdruck zugeordnet. Es gibt diverse Weiterentwicklungen dieses Verfahrens, die dem Grunde nach aber alle auf dem gleichen Grundprinzip der Druckmessung und Messung der Druckschwankungen beruhen.

Der Nachteil bei diesen Verfahren ist das Empfinden des Patienten. Das Aufpumpen der Manschette ist für den Patienten unangenehm und kann bei höheren Manschettendrücken sogar schmerzhaft werden. Zusätzlich läßt sich die Messung nicht permanent wiederholen, da eine Schädigung der Arterie auftreten kann, so dass die kontinuierliche Erfassung des Blutdruckes nachteilig nicht möglich ist. Es müssen zwingende Meßpausen eingelegt werden. Zusätzlich ist der Vorgang des Aufpumpens und Ablassens der Luft in der Manschette zeitaufwendig und umständlich. Eine gewünschte kontinuierliche Erfassung von Messwerten und Daten zu Blutwerten ist mit den bekannten Verfahren nicht möglich. Es ist allerdings wünschenswert, den Blutdruck und andere Vitalwerte und Körperinhaltsstoffe nebeneinander und zusätzlich möglichst permanent und kontinuierlich zu erfassen.

Bei den kontinuierlichen, Verfahren wie zum Beispiel der Volumenkompensationsmethode und der arteriellen Applantationstonometrie, sind andere Nachteile mit dem Messverfahren verbunden. Bei der Volumenkompensationsmethode treten Schmerzen beim Patienten auf, insbesondere bei der Messung am Finger. Zusätzlich treten bei eingeschränkter peripherer Durchblutung wie zum Beispiel bei Schock oder schockartigen Zuständen Phänomene auf, welche die Messung des Blutdrucks verfälschen und eine ungenaue Messung bedingen. Teilweise ist aber gerade bei solchen Patienten, insbesondere in kritischen Phasen, eine möglichst genaue und einfache sowie schnelle Erfassung möglichst vieler Messdaten, wie Blutdruck und Vitalwerten wünschenswert und notwendig. Bei der arteriellen Applantationstonometrie sind aber Bewegungsartefakte hinderlich bezüglich den absoluten Messwerten und der daraus resultierenden Messgenauigkeit. Die Messergebnisse sind eher unbefriedigend, sofern der Patient nicht völlig ruhig gestellt ist. Das regelmäßige Nachjustieren und das neue Kalibrieren der Messvorrichtung wird bereits bei geringen Verschiebungen der Sensoren an der Patientenschnittstelle notwendig.

Allen bekannten Verfahren liegt jedoch neben der Problematik der Artefakte auch das gemeinsame Problem zugrunde, dass die benötigten Werte nur isoliert gemessen werden können. Es fehlen zeitlich korrelierte Angaben zu chemischen Konzentrationswerten des Blutes, insbesondere Werte aus der nicht-invasiven Bestimmung von Vitalparametern und biophysikalischen Größen wie Blutzucker und anderer elementarer Bestandteile des Blutes oder der zu untersuchenden Körperflüssigkeiten. Unabhängig vom Blutdruck kann es auch notwendig sein ausschließlich die Vitalwerte zu bestimmen, wozu die bekannten Verfahren, die auf die alleinige Bestimmung von Blutdruckwerten ausgerichtet sind, ungeeignet sind.

Nicht-invasive Verfahren zur Bestimmung von Vitalwerten und Inhaltsstoffen von Flüssigkeiten sind ebenfalls im Stand der Technik bekannt. Besonders hervorzuheben ist dabei die Messung des Sauerstoffsättigungspegels.

Dabei wird Licht im sichtbaren und nahen Infrarotbereich des elektromagnetischen Spektrums für Messungen der Sauerstoffsättigungspegel von Patientenblut verwendet. Hierzu werden spektrometrische Instrumente in Verbindung mit geeigneten, üblicherweise mehreren Sensorflächen verwendet, mittels welcher die Sauerstoffsättigung von Blut abgeschätzt bzw. näherungsweise berechnet wird.

Es sind unterschiedliche Verfahren der Pulsoximetrie bekannt, um den Sauerstoffsättigungswert von arteriellem Blut möglichst genau zu bestimmen.

Es ist weiterhin bekannt, dass die arterielle Sauerstoffsättigung durch Isolieren der Veränderung in den detektierten Lichtintensitäten während eines Herzzyklus und dem Versuch, die Absorptionseffekte von nicht-arteriellem Blutgewebe des Patienten zu minimieren und sogar zu eliminieren, ermittelt werden kann. Obwohl diese Art der Oximetriemessung versucht, eine Vielzahl von Artefakte wie Einflüsse von Knochen, Haut und Muskeln u.s.w. zu eliminieren, besteht der Nachteil darin, dass die Signalaufnahmeschaltkreise und die Analyseschaltkreise sehr robust ausgeführt sein müssen, da der verwendbare und verwertbare Teil des Mess-Signals die relativ kleine Veränderung der ermittelten und detektierten Intensitäten ist und diese klein ist bezogen auf die gesamte detektierte Intensität. Zusätzlich werden die Messergebnisse durch pulsierende Signalbeiträge von vielen nahe gelegenen Gewebeschichten beeinflußt, so dass der ermittelte Messwert der Sauerstoffsättigung eher ungenau ist und verfälscht sein kann.

Es sind weitere Verfahren der Spektralanalyse und der differentiellen optischen Bestimmung des Sauerstoffgehaltes des menschlichen Blutes bekannt, die alle jedoch die Nachteile von Artefakten aufweisen und keinen Beitrag zur Erfassung des Blutdrucks oder zu anderen chemischen Inhaltsstoffen des Blutes liefern. Ein weiterer Nachteil besteht darin, dass die genannten Messverfahren nicht geeignet sind gleichzeitig den Blutdruck des Patienten oder der zu untersuchenden Person zu erfassen. Hierzu wäre ein paralleles und zusätzlich zeitlich aufwendiges Verfahren notwendig, um den Blutdruck zu messen. Weitere Vitalwerte können ebenfalls mit den bekannten Messeinrichtungen und hier beschriebenen Verfahren nicht ermittelt werden.

Allen bekannten Verfahren und Vorrichtungen gemeinsam sind die Nachteile von Messungenauigkeiten, dem Einfluß von Artefakte und insbesondere dem Nachteil, dass es nicht möglich ist, unterschiedliche Inhaltsstoffe mit einem identischen Verfahren ermitteln zu können. Daneben sind einige Verfahren der Blutdruckmessung schmerzhaft und unangenehm.

In der Medizin und bei der Patientenüberwachung ist die permanente Kontrolle des Blutdrucks und das wiederholte Ermitteln von Blutwerten und Körperinhaltsstoffen häufig von erheblicher Bedeutung.

Das vorliegende Verfahren versucht neben den oben genannten Nachteilen auch weitere Nachteile etablierter Verfahren zu umgehen. Beispielsweise ist das Eliminieren der Belastungen für den Patienten gegenüber der invasiven Entnahme von Körperflüssigkeiten wie Blut und gegenüber dem Infektrisiko, welches ein latentes Risiko bei den etablierten invasiven Verfahren darstellt, ein erheblicher Vorteil und bietet darüber hinaus das Potential zur Verringerung des für eine Messung erforderlichen Zeitbedarfs sowie der damit verbundenen Kosten.

Darüber hinaus werden häufig nicht nur einzelne Werte wie der Blutdruck oder Natriumgehalt des Blutes zu ermitteln sein, sondern es werden zusätzlich eine ganze Reihe von Messdaten benötigt, so dass diese mit den traditionellen etablierten Verfahren nacheinander oder mit unterschiedlichen Messeinrichtungen nebeneinander ermittelt werden müssen. Nachteilig wirkt sich dabei auch das Tauschen der Messeinrichtungen auf die Referenzmesswerte aus.

Daher liegt der vorliegenden Erfindung die Aufgabe zugrunde, ein Verfahren und eine Vorrichtung zur verbesserten nicht-invasiven Messung von Körperinhaltsstoffen und Blutmeßwerten sowie dem Blutdruck bereitzustellen. Diese Aufgabe wird vorrichtungsartig durch die Merkmale des Anspruchs 1 und verfahrensseitig durch die Merkmale des Anspruchs 21 gelöst.

Die Erfindung geht von der Grunderkenntnis aus, dass die elektro-chemischen Vorgänge im menschlichen Herzen und Körper und infolgedessen die konkrete chemische Zusammensetzung des Blutes einer zu untersuchenden Person oder eines Patienten in direktem Zusammenhang und damit in einer meßbaren Abhängigkeit zum arteriellen Blutdruck des Patienten stehen bzw. sich mit diesem entsprechend ändern. Insbesondere verhält sich die lokale Konzentration von chemischen Elementen des Blutes abhängig vom tatsächlich vorhandenen arteriellen Blutdruck. Beispielsweise ändert sich konkret der Natrium- und Kaliumanteil in Korrelation zum Blutdruck. Dieser Zusammenhang zwischen der Konzentration von Inhaltsstoffen zum arteriellen Blutdruck ist von prinzipieller Natur.

Weiterhin geht die Erfindung von den physikalischen Grundlagen und den Gesetzmäßigkeiten bei der Ausbreitung von elektromagnetischen Wellen insbesondere von den Grundlagen bei den auftretenden Wechselwirkungen der Lichtwellen im sichtbaren, UV-und Infrarotbereich bei Durchdringung von flüssigen Körpern aus. Hierzu sei das Lambert-Beersche-Gesetz erwähnt, welches einerseits den Zusammenhang von der Gesamtabsorption eines Mediums in Abhängigkeit der Konzentration der enthaltenen Substanzen beschreibt und andererseits auf das Phänomen eingeht, dass es einen mathematischen Zusammenhang zwischen der emittierten und absorbierten Lichtintensität einer Lichtwelle, die eine solche Substanz durchläuft, gibt.

Die Vorrichtung und das Verfahren der vorliegenden Erfindung basieren auf dem Prinzip der spektroskopischen Sensorik unter Verwendung geeigneter schmalbandiger, durchstimmbarer Lichtquellen oder ganz allgemein einer schmalbandigen, durchstimmbaren Aktorik.

Die Erfindung kombiniert eine optisch durchstimmbare Aktorik, insbesondere eine optisch durchstimmbare Lichtquelle, mit einer optisch durchstimmbaren Sensorik, insbesondere einem optisch durchstimmbaren Empfänger. Erfindungsgemäß wird zur Ermittlung der gewünschten Meßdaten der komplette sichtbare und nicht-sichtbare Frequenzbereich des optischen Spektrums in der optisch durchstimmbaren Aktorik genutzt, um mittels eines optischen Verfahrens die gewünschten Meßwerte in der optischen Sensorik zu erhalten. In einer vorzugsweisen Ausführungsform werden Aktorik und Sensorik bzw. Sender und Empfänger mittels einer Steuer- und Regeleinheit zueinander synchronisiert. Neben einer einzelnen Aktorik oder Sensorik können auch mehrere Sender und Empfänger oder ein Sender mit mehreren Empfängern sowie mehrere Sender mit einem Empfänger jeweils in der Aktorik oder der Sensorik miteinander kombiniert und entsprechend der Messaufgabe zueinander in geeigneter Weise synchronisiert werden.

Dabei wird mittels des optisch durchstimmbaren Senders der Aktorik das zu untersuchende Blut an der Patientenschnittstelle (zum Beispiel Finger, geeignete Körperpartie oder Gewebe) des Patienten vorzugsweise mit monochromatischer elektromagnetischer Strahlung durchleuchtet. Der optisch durchstimmbare Sender kann dabei eine im geforderten Spektralbereich durchstimmbare, schmalbandige Lichtquelle sein und der Empfänger kann ein im entsprechenden Spektralbereich durchstimmbarer optischer Spektrumanalysator sein. Die Auswertung der spektralen Linien der optischen Aktorik und Sensorik ergibt unter Zuhilfenahme einer geeigneten Auswerteanalytik die gesuchten Messwerte der untersuchten Blutbestandteile oder der untersuchten Körperflüssigkeiten, die sich aus der Validierung der detektierten Empfangssignale im Empfänger für den jeweiligen Patienten ergeben. Um frei von Artefakten zu sein kann zusätzlich ein Kalibrierprozess der Mess-Amplituden durchgeführt werden. Durch die Synchronisation und die Kenntnis um die Normalamplitude bezogen auf die gemessene Lichtintensität läßt sich ein rechnerischer Multiplikator finden, der aus dem Verhältnis der gemessenen Amplitude zu der Normalamplitude resultiert, um mit diesem einen Kalibrierungsprozess durchzuführen. Bewegungen und andere bewegungsbedingte Artefakte werden infolge der Synchronisation der Sende-Empfangsstrecke eliminiert.

Alternativ können neben den bereits erwähnten Körperflüssigkeiten oder dem Blut auch geeignete Gewebeteile genutzt werden, um das erfindungsgemäße Verfahren anzuwenden und die gewünschten Vitalwerte, Körperinhaltsstoffe und die benötigten Meßwerte zu ermitteln.

Eine Vorrichtung, welche das Verfahren der vorliegenden Erfindung kombiniert besteht daher aus einer optischen Sendevorrichtung und einer optischen Empfangsvorrichtung sowie einer Auswertevorrichtung, vorzugsweise einer elektronischen Auswertevorrichtung, welche die Zusammenhänge der emittierten Spektrallinien des Senders und die der detektierten Spektrallinien in der Empfangseinrichtung des Empfängers entsprechend geeigneter mathematischer Methoden validiert und als numerische Werte ausgibt. Eine mögliche Ausgabeform ist die der digitalen Anzeigen der gemäß dem Kalibrierverfahren errechneten Messwerte. Alternativ können auch Schnittstellen bereitgestellt werden, welche die ermittelten Signale vom Empfänger in gewünschte Ausgabeformate umrechnen und beispielsweise in Form von graphischen Tabellen oder Graphiken ausgeben.

Weitere Ausführungsformen sind den weiteren abhängigen Ansprüchen zu entnehmen.

Im Folgenden werden bevorzugte Ausführungsformen der Erfindung unter Bezugnahme auf die Figuren der Zeichnung näher beschrieben.
- Fig. 1: zeigt ein schematisches Blockschaltbild, welches das zugrunde liegende Verfahren und schematisch eine Vorrichtung zur Ermittlung von Körperinhaltsstoffen und Blutwerten illustriert;
- Fig. 2: zeigt ein der Fig.1 ähnliches schematisches Blockschaltbild, jedoch mit zwei optisch durchstimmbaren Sendern und zwei optisch durchstimmbaren Empfänger sowie einer Auswertevorrichtung;
- Fig. 3: zeigt ebenfalls ein schematisches Blockschaltbild ähnlich der Fig.1 und 2, jedoch mit einem optischen Prisma;
- Fig. 4: zeigt den Zusammenhang einer Medikamention auf die detektierte Spektrallinie nach der Absorption;
- Fig. 5: zeigt die prinzipielle spektrale Verteilung von verschiedenen Stoffen in Bezug auf die Wellenlänge und deren charakteristischen Absorptionsbanden;
- Fig. 6: zeigt das Spektrum des Lichtes mit dem für das vorliegende Verfahren notwendigen, durchstimmbaren Wellenlängenbereich;
- Fig. 7: zeigt exemplarisch die Merkmale einer idealen durchstimmbaren Lichtquelle mit einer Spektralbande; und
- Fig. 8: zeigt schematisch das Schaltbild ähnlich der Fig.1 mit einem Finger als Beispiel einer Patientenschnittstelle.

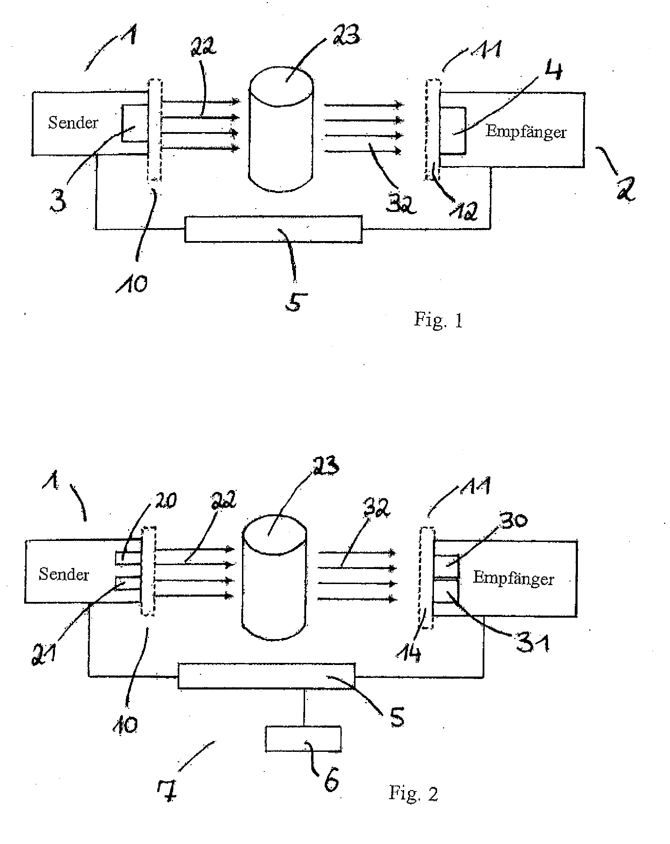

Fig.1 zeigt eine Sendeeinrichtung 1, welche eine geeignete durchstimmbare Lichtquelle in Form eines optisch durchstimmbaren Senders 3 aufweist. Der Sender 3 verfügt über eine erste Einrichtung 10, beispielsweise einen Monochromator zur Erzeugung von monochromatischem Licht in Form von elektromagnetischer Strahlung 22, welches im optisch durchstimmbaren Sender 3 erzeugt wird. Der Sender 3 ist derart zu einer Patientenschnittstelle 23 orientiert, dass die aus dem durchstimmbaren Sender austretende, monochromatische elektromagnetische Strahlung in die Patientenschnittstelle 23 eindringt und diese wie im vorliegenden Beispiel passiert bzw. daran gestreut wird. Dabei kommt es zu einer gewünschten und für jede Substanz signifikanten Wechselwirkung mit den zu messenden Inhaltsstoffen in Abhängigkeit von deren Vorhandensein und deren Konzentration. Die aus der Patientenschnittstelle austretende, im vorliegenden Fall transmittierte Strahlung 32 wird in einer Empfangseinrichtung 2 von einem optisch durchstimmbaren Empfänger 4 erfasst. Der optisch durchstimmbare Sender 3 und der optisch durchstimmbare Empfänger 4 sind über eine Steuer- oder Regelungseinheit 5 zueinander synchronisiert. Mit dem Bezugszeichen 11 ist eine Einrichtung bezeichnet, welche den Empfänger 4 stufenlos durchstimmt. Im vorliegenden Fall wurde beispielsweise ein optisches Gitter 12 verwendet.

Fig. 2 zeigt ein zweites Ausführungsbeispiel mit einem ähnlichen Aufbau wie in Fig.1. In diesem Ausführungsbeispiel hat jedoch der Sender 3 zwei getrennte Lichtquellen, die jeweils parallel als optisch durchstimmbare Sender 20, 21 arbeiten können. Eine hierzu vergleichbare Anordnung ist auf der Empfängerseite dargestellt. Hier sind zwei optisch durchstimmbare Empfänger 30, 31 voneinander getrennt dicht nebeneinander angeordnet.

Das Verfahren kann je nach Messaufgabe auch nur mit einem der beiden Empfänger durchgeführt werden. Die dargestellten Empfänger 30, 31 können beispielsweise optisch durchstimmbare schmalbandige Spektralanalysatoren sein. Die Steuer- oder Regelungseinheit 5, welche die Sender 20, 21 mit den Empfängern 30, 31 entweder paarweise synchronisiert oder wahlweise den Sender 20, 21 beispielsweise mit einem Empfänger 30 synchronisiert, bietet dadurch die Möglichkeit auch absolut differentielle Signale zueinander auszuwerten und die empfangenen Signale von zwei gleichzeitig ausgesendeten Lichtfrequenzen der Sender 20, 21 miteinander zu vergleichen. Unter Ausnutzung des Dopplereffektes lassen sich mit einer Auswerteeinrichtung 6 zum Beispiel strömende Stoffe in der Patientenschnittstelle 23 mittels des differentiellen Verfahrens bestimmen.

Die Auswerteeinrichtung 6 dient der Kalibrierung, Auswertung und Validierung der ermittelten Messwerte auf Basis von mathematischen Methoden. Die Aufgabe der Einrichtung (11) aus Fig.1 übernimmt hier ein elektronischer schmalbandiger Filter (14). Es ist denkbar die Steuer- oder Regelungseinheit 5 und die Auswerteeinrichtung 6 in einer gemeinsamen Regel- und Auswertevorrichtung 7 unterzubringen.

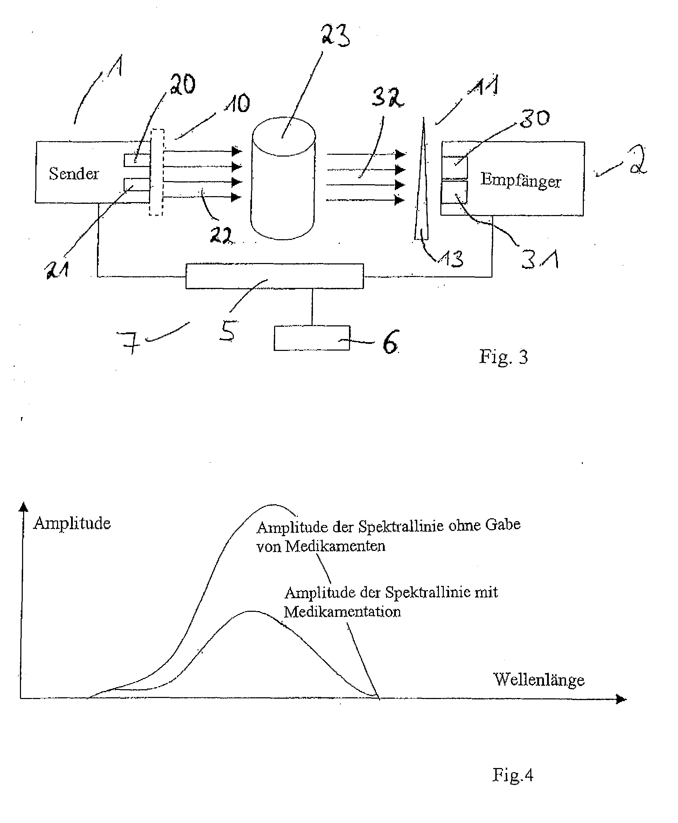

Fig. 3 zeigt ein drittes Ausführungsbeispiel ähnlich dem aus Fig. 2. Hier befindet sich in Verbindung zu den Empfängern 30, 31 dargestellt ein optisches Prisma 13, welches eine alternative Möglichkeit neben einem Beugungsgitter der Einrichtung 11 aus Fig. 2 darstellt.

Die in Fig. 4 gezeigte Grafik gibt den Zusammenhang zwischen einer erfassten Lichtfrequenz bestimmter Intensität und der Amplitude der Spektrallinie (obere Kurve) bei Nichtvorhandensein eines Medikamentes an. Hier fehlt die Medikation, so dass daher keine Absorptionseffekte und Wechselwirkungen in der Patientenschnittstelle 23 mit bestimmten auf das Medikament ansprechenden Inhaltsstoffen (bsp. Kaliumkonzentration) auftreten in einem dafür signifikanten Wellenlängenbereich. Das bevorzugte Verfahren detektiert jedoch mittels des synchron auf den Sender abgestimmten Empfängers nach Verabreichung von Medikamenten ein niedrigeres Signal nach Durchlaufen der Patientenschnittstelle 23 (bsp. des Fingers eines Probanten) innerhalb des dafür signifikanten Wellenlängenbereich (untere Kurve) .

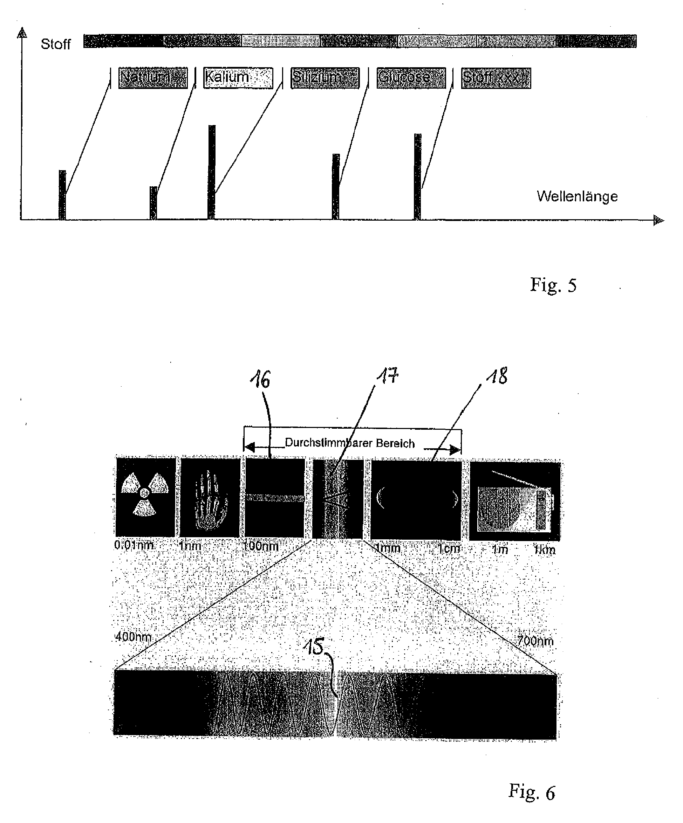

Chemische Stoffe und Elemente zeigen eine für diese Stoffe und Elemente typische spektrale Verteilung. In der Fig. 5 ist exemplarisch für die Stoffe Natrium, Kalium, Silizium, Glucose und einem weiteren beispielhaften Stoff die prinzipielle spektrale Verteilung in Abhängigkeit von der Wellenlänge dargestellt. Die Höhe der Amplitude ist dabei unterschiedlich und abhängig von diversen Faktoren, wie beispielsweise der eingestrahlten Lichtleistung und in Abhängigkeit von der Konzentration der betroffenen Stoffe.

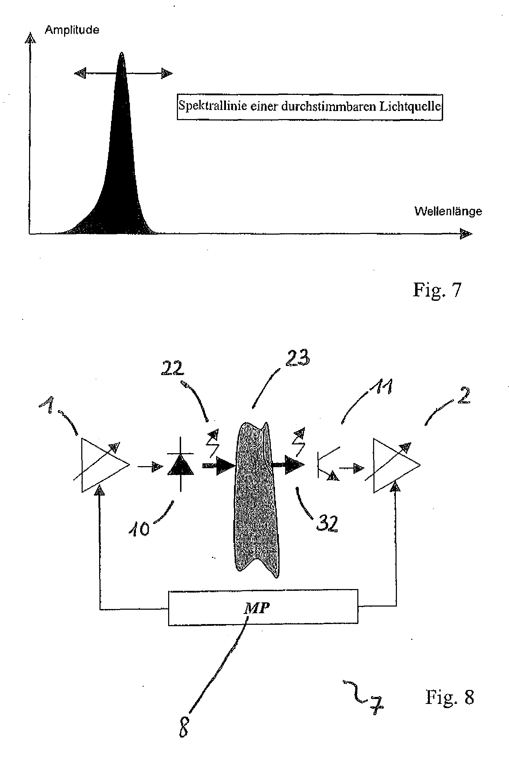

Beispielhaft ist in Fig. 6 das Lichtspektrum gezeigt und gegen die Wellenlänge aufgetragen. Der für diese Erfindung vorzugsweise genutzte Spektralbereich lässt sich anhand der Fig. 6 in die drei folgenden Bereiche einteilen, über welche man die optischen Sender 3, 20, 21 und die optischen Empfänger 4, 30, 31 durchstimmen kann: UV-Bereich 16, sichtbarer Bereich 17 und IR-Bereich 18. Die Vorzugswerte liegen etwa zwischen 100 nm und 1 cm. Der Begriff der durchstimmbaren Lichtquelle wird in der Fig. 7 näher erläutert. Hier ist die Amplitude einer beliebigen Spektrallinie über die Wellenlänge aufgetragen. Die horizontalen und vertikalen Pfeile geben die Bereiche der durchstimmbaren Faktoren wie Amplitude und Frequenz bzw. Wellenlänge an.

In Fig. 8 ist eine Schaltanordnung ähnlich der in Fig. 1 dargestellt. Es kann vorzugsweise zur Bewältigung der auftretenden hohen Datenmengen und der dadurch notwendigen hohen Rechengeschwindigkeit und gleichzeitig zur schnellen Nachführung bei der Synchronisation der Sender-Empfängerstrecke ein Mikroprozessor 8 zu dieser Aufgabe verwendet werden.

### Bezugszeichenliste:

- 1: Sendeeinrichtung
- 2: Empfangseinrichtung
- 3: optisch durchstimmbarer Sender
- 4: optisch durchstimmbarer Empfänger
- 5: Steuer- oder Regelungseinheit
- 6: Auswerteeinrichtung
- 7: Regel- und Auswertevorrichtung
- 8: Mikroprozessor
- 10: erste Einrichtung
- 11: zweite Einrichtung
- 12: optisches Gitter
- 13: optisches Prisma
- 14: elektronischer schmalbandiger Filter
- 15: Frequenzbereich
- 16: UV-Frequenzbereich
- 17: sichtbarer Frequenzbereich
- 18: Infrarotfrequenzbereich (IR-Frequenzbereich)
- 20: optisch durchstimmbarer Sender bzw. optischer Sender
- 21: optisch durchstimmbarer Sender bzw. optischer Sender
- 22: Elektromagnetische Strahlung
- 23: Patientenschnittstelle
- 30: optisch durchstimmbarer Empfänger bzw. optischer Empfänger
- 31: optisch durchstimmbarer Empfänger bzw. optischer Empfänger
- 32: transmittierte Strahlung

## Patentansprüche

1. Vorrichtung zur nicht-invasiven, optischen Erfassung von chemischen und physikalischen Blutwerten und Körperinhaltsstoffen an einer Patientenschnittstelle (23), mit mindestens einer Sendeeinrichtung (1) in Form eines optisch durchstimmbaren Senders (3) und mindestens einer zugeordneten Empfangseinrichtung (2) in Form eines optisch durchstimmbaren Empfängers (4),
**dadurch gekennzeichnet, dass**
der mindestens eine optisch durchstimmbare Sender (3) und der mindestens eine zugeordnete optisch durchstimmbare Empfänger (4) mittels einer zugeordneten Steuer- oder Regelungseinheit (5) zueinander synchronisierbar sind.

2. Vorrichtung gemäß Anspruch 1, **dadurch gekennzeichnet,**
**dass** der Blutdruck mittels detektierter Daten im optisch durchstimmbaren Empfänger (3) ermittelbar ist.

3. Vorrichtung gemäß Anspruch 1 oder 2, **dadurch gekennzeichnet, dass** die Steuer- oder Regelungseinheit (5) mit einer Auswerteeinrichtung (6) zu einer Regel- und Auswertevorrichtung (7) zusammengefasst sind.

4. Vorrichtung gemäß einem der vorhergehenden Ansprüche, **dadurch gekennzeichnet, dass** der optische Sender (3) mittels einer ersten Einrichtung (10) stufenlos über einen Frequenzbereich (15) durchstimmbar ist.

5. Vorrichtung gemäß einem der vorhergehenden Ansprüche, **dadurch gekennzeichnet, dass** der optische Empfänger (4) über eine zweite Einrichtung (11) stufenlos über einen Frequenzbereich (15) durchstimmbar ist.

6. Vorrichtung gemäß Anspruch 4 oder 5, **dadurch gekennzeichnet, dass** der optische Sender (3) mittels der ersten Einrichtung (10) vom UV-Frequenzbereich (16) über den sichtbaren Frequenzbereich (17) zum Infrarot-Frequenzbereich (18) stufenlos durchstimmbar ist.

7. Vorrichtung gemäß Anspruch 5 oder 6, **dadurch gekennzeichnet, dass** der optische Empfänger (4) mittels der zweiten Einrichtung (11) vom UV-Frequenzbereich über den sichtbaren Frequenzbereich zum Infrarot-Frequenzbereich stufenlos durchstimmbar ist.

8. Vorrichtung gemäß einem der vorhergehenden Ansprüche, **dadurch gekennzeichnet, dass** der optische Empfänger (4) mittels eines optischen Gitters (12) über einen Frequenzbereich stufenlos durchstimmbar ist.

9. Vorrichtung gemäß einem der vorhergehenden Ansprüche, **dadurch gekennzeichnet, dass** der optische Empfänger (4) mittels eines optischen Prismas (13) über einen Frequenzbereich stufenlos durchstimmbar ist.

10. Vorrichtung gemäß einem der vorhergehenden Ansprüche, **dadurch gekennzeichnet, dass** der optische Empfänger (4) mittels elektronischer, schmalbandiger Filter (14) über einen Frequenzbereich stufenlos durchstimmbar ist.

11. Vorrichtung gemäß einem der vorhergehenden Ansprüche, **dadurch gekennzeichnet, dass** der optische Sender (3) ein schmalbandiges nahezu monochromatisches Licht aussendet.

12. Vorrichtung gemäß einem der vorhergehenden Ansprüche, **dadurch gekennzeichnet, dass** der optische Sender (3) kohärentes Licht aussendet.

13. Vorrichtung gemäß einem der vorhergehenden Ansprüche, **dadurch gekennzeichnet, dass** der optische Sender (3) zusätzlich zu unpolarisiertem Licht polarisiertes Licht aussendet.

14. Vorrichtung gemäß einem der vorhergehenden Ansprüche, **dadurch gekennzeichnet, dass** der Sender (3) zwei oder mehrere optische Sendeeinheiten bzw. getrennte, optisch durchstimmbare Sender (20, 21) aufweist.

15. Vorrichtung gemäß einem der vorhergehenden Ansprüche, **dadurch gekennzeichnet, dass** der Empfänger (4) mehrere optische Empfangseinheiten bzw. getrennte, optisch durchstimmbare Empfänger (30, 31) aufweist.

16. Vorrichtung gemäß Anspruch 14 oder 15, **dadurch gekennzeichnet, dass** die optischen Sender (20, 21) zu mindestens einem der beiden optischen Empfänger (30, 31) mittels der Steuer- oder Regelungseinheit (5) jeweils synchronisiert sind.

17. Vorrichtung gemäß Anspruch 16, **dadurch gekennzeichnet, dass** die Steuer- oder Regelungseinheit (5) mittels eines differentiellen Analyseverfahrens die chemischen und physikalischen Werte der Körperinhaltstoffe und des Blutdruckes aus den detektierten differentiellen Signalen mindestens eines optisch durchstimmbaren Empfängers (30, 31) aus unterschiedlichen Signalen der optisch durchstimmbaren Sender (20, 21) ermittelt.

18. Vorrichtung gemäß Anspruch 17, **dadurch gekennzeichnet, dass** die Regel- und Auswerteeinheit (7) unter Zuhilfenahme der Grundlagen des Dopplereffektes zusätzlich Werte zum Strömungsverhalten innerhalb der Körperflüssigkeiten ermittelt.

19. Vorrichtung gemäß einem der vorhergehenden Ansprüche, **dadurch gekennzeichnet, dass** die Synchronisation des/der optisch durchstimmbaren Sender/s (3, 20, 21) und des/der optisch durchstimmbaren Empfänger/s (4, 30, 31) und die Validierung der ermittelten Signale über eine Steuereinheit (5) und einen Mikroprozessor (8) erfolgt.

20. Vorrichtung gemäß einem der vorhergehenden Ansprüche, **dadurch gekennzeichnet, dass** der Empfänger (4) ein Spektrumanalysator mit integrierter Auswerteeinrichtung (6) ist.

21. Verfahren zur nicht-invasiven, optischen Erfassung von chemischen und physikalischen Blutwerten und Körperinhaltsstoffen an einer Patientenschnittstelle (23) mittels mindestens einem optisch durchstimmbaren Sender (3, 20, 21) und mindestens einem zugeordneten optisch durchstimmbaren Empfänger (4, 30, 31) unter Verwendung einer Steuer- oder Regelungseinheit (5) zum Synchronisieren des mindestens einen Senders (3, 20, 21) und des mindestens einen zugeordneten Empfängers (4, 30, 31) zueinander, mit folgenden Verfahrensschritten:
a) Einbringen einer schmalbandigen, elektromagnetischen Strahlung in die Patientenschnittstelle (23) mittels des mindestens einen optisch durchstimmbaren Senders (3, 20, 21);
b) Validieren der nach dem Passieren der Patientenschnittstelle (23) detektierten Signale in dem mindestens einen zugeordneten optisch durchstimmbaren Empfänger (4, 20, 21); und
c) Kalibrieren der ermittelten Messdaten mittels eines Kalibrierungsprozesses.

22. Verfahren gemäß Anspruch 21, **dadurch gekennzeichnet, dass** mittels der Steuer- oder Regelungseinheit (5) aus den in der Empfangseinheit (4) detektierten Signalen und den daraus resultierenden Konzentrationswerten von Körperinhaltsstoffen der korrelierte arterielle Blutdruck validiert und ermittelt wird.

23. Verfahren gemäß Anspruch 21 oder 22, **dadurch gekennzeichnet, dass** das Verfahren mittels mindestens eines synchronisierten und optisch durchstimmbaren Senders (3) und mindestens eines zugeordneten, synchronisierten und optisch durchstimmbaren Empfängers (4) unter Verwendung einer Vorrichtung mit den Merkmalen gemäß einem der Ansprüche 1 bis 20 durchgeführt wird.
